# EUROPÄISCHE PATENTSCHRIFT

(11) **EP 2 524 672 B1**
(45) Veröffentlichungstag und Bekanntmachung des Hinweises auf die Patenterteilung: **10.04.2019**
(21) Anmeldenummer: 12168152.2
(22) Anmeldetag: 16.05.2012
(51) Int. Cl.: A61F 5/01, A61F 2/64

(54) **Gelenk für Kniegelenks-Orthesen, -Prothesen bzw. -Stützen**
Joint for knee orthotics, prosthetics and supports
Articulation pour soutiens, orthèses ou prothèses du genou

(30) Priorität: 19.05.2011 AT 7182011
(43) Veröffentlichungstag der Anmeldung: 21.11.2012
(73) Patentinhaber: Wayd, Kurt, 1020 Wien (AT)
(72) Erfinder: Wayd, Kurt, 1020 Wien (AT); Grafinger, Josef, 1160 Wien (AT); Frass, Albert, 2340 Mödling (AT)
(74) Vertreter: Patentanwälte Pinter & Weiss OG

(56) Entgegenhaltungen:
- EP-A1- 0 925 766
- WO-A1-2009/098724
- US-A1- 2003 100 853
- US-A1- 2005 148 916
- US-A1- 2005 192 523

## Beschreibung

Die Erfindung betrifft ein Gelenk für Kniegelenks-Orthesen, -Prothesen bzw. -Stützen, mit zumindest einem dem Unterschenkel zugeordneten ersten Gelenkschenkel und zumindest einem dem Oberschenkel zugeordneten zweiten Gelenkschenkel, welche mit ihren Enden derart überlappend verbunden sind, dass bei zunehmendem Winkel zwischen den Gelenkschenkeln eine kombinierte Roll- und Gleitbewegung in der durch die beiden Gelenkschenkel definierten Ebene bewirkt ist.

Die Begriffe "Drehen", "Rollen" und "Gleiten" werden im Zusammenhang mit den in einem Kniegelenk stattfindenden Bewegungen unterschiedlich interpretiert. Eine Drehbewegung ist im technischen Sinne gewöhnlich die Drehung eines Körpers um eine feststehende Achse. Eine Rollbewegung ist im technischen Sinne vergleichbar mit einem Rad welches auf einer ebenen Fläche rollt, wobei sich das Zentrum des Rades in Richtung der Rollbewegung verschiebt. Eine Gleitbewegung ist im technischen Sinne die Verschiebung eines Körpers relativ zu einem anderen Körper oder seiner Unterlage, vergleichbar mit einem blockierenden Rad welches auf einer ebenen Fläche geschoben wird (z.B. Bremsspur).

Eine gleichzeitige Roll- und Gleitbewegung wie sie im Kniegelenk vorkommt, ist in diesem Sinne vergleichbar mit einem Rad welches auf einer ebenen Fläche zwar rollt, wobei sich das Drehzentrum jedoch in Richtung der Bewegung um einen kleineren oder höheren Wert verschiebt als es mathematisch der Rollbewegung entsprechen würde (analog dem Durchdrehen bzw. Blockieren der Räder eines Fahrzeuges).

US 2005/0148916 A1 und WO 2009/098724 A1 offenbaren jeweils Gelenke für Kniegelenksorthesen. Die Gelenke weisen jeweils zwei flache, sich in einem Gelenksbereich überlappende Gelenksschenkel auf, wobei auf einem Gelenksschenkel Führungselemente abstehend angeordnet sind, die in Führungsbahnen, die in dem anderen Gelenksschenkel ausgearbeitet sind, eingreifen und darin geführt sind, um die Beugebewegung des menschlichen Knies nachzuahmen.

Die Aufgabe der vorliegenden Erfindung war ein Gelenk der eingangs angegebenen Art, welches durch ein variables Rotationszentrum die Kinematik des natürlichen menschlichen Knies optimal nachempfindet.

Zur Lösung dieser Aufgabe ist die Erfindung dadurch gekennzeichnet, dass im ersten Gelenkschenkel eine erste, im Wesentlichen gerade, nutartige Ausnehmung im Wesentlichen in Richtung der Längsachse des Gelenkschenkels orientiert ausgearbeitet ist, dass weiters im ersten Gelenkschenkel ein im Wesentlichen gerader Längsschlitz mit geringerer Breite als die Ausnehmung und im Wesentlichen senkrecht auf diese Ausnehmung ausgearbeitet ist, und dass am zweiten Gelenkschenkel zwei im Wesentlichen kreisrunde Führungselemente angesetzt sind, wobei das dem Gelenkschenkel unmittelbar anliegende Führungselement exzentrisch angeordnet und mit einem der Breite der Ausnehmung entsprechenden Durchmesser versehen ist, und wobei das zweite Führungselement auf dem ersten Führungselement aufgesetzt ist, einen der Breite des Längsschlitzes entsprechenden Durchmesser aufweist und im Wesentlichen im Zentrum des mit dem anderen Gelenkschenkel überlappenden Endes des Gelenkschenkels angeordnet ist, wobei bei zunehmendem Winkel zwischen den Gelenkschenkeln eine kombinierte Roll- und Gleitbewegung des zweiten Gelenkschenkels in der durch die beiden Gelenkschenkel definierten Ebene in einer Richtung bewirkt ist, welche im wesentlichen radial zur Achse des ersten Gelenkschenkels in den zwischen den beiden Gelenkschenkel eingeschlossenen Quadranten führt, wobei der Drehpunkt der beiden Gelenkschenkel für einen Winkel zwischen den Gelenkschenkeln zwischen 0 und 25° im Wesentlichen stationär bleibt. Damit ist bei relativ einfacher und kostengünstiger Herstellung der beiden Gelenkschenkel eine orthopädisch optimale Anpassung der relativen Bewegung der Gelenkschenkel zueinander während der Beuge- bzw. Streckbewegung möglich.

Gemäß einer hierin offenbarten Entwicklung, die kein Gegenstand der Erfindung ist, ist eine erste Kurve als im wesentlichen gerader Schlitz und eine zweite Kurve um den Schlitz als Zentrum gekrümmt im ersten Gelenkschenkel ausgearbeitet, wobei die Verschiebung des Führungselementes in der zweiten Kurve bei zunehmendem Winkel eine vorzugsweise progressive Verschiebung des anderen Führungselementes im Schlitz bewirkt.

Vorteilhafterweise sind dabei in einem der Gelenkschenkel zwei Kurven in Schlitzform ausgearbeitet, deren relativer Verlauf bei zunehmendem Winkel zwischen den Gelenkschenkeln eine Verschiebung des zweiten Gelenkschenkels im wesentlichen radial zur Achse des ersten Gelenkschenkels in das zwischen den beiden Gelenkschenkel eingeschlossene Kreissegment bewirkt, wobei die beiden Kurven vorzugsweise gekrümmt und mit ihren konkaven Seiten einander zugewandt sind.

Eine andere hierin offenbarten Entwicklung, die kein Gegenstand der Erfindung ist, sieht vor, dass einer der Gelenkschenkel einen Hohlkörper aufweist, in welchen der andere Gelenkschenkel hineinragt, wobei die innere Oberfläche des Hohlkörpers zumindest zwei Kurven aufweist, an welchen zumindest zwei nockenförmige Aussenkonturen des anderen Gelenkschenkels als Führungselemente anliegen.

Um zusätzlich eine Anpassung an die jeweilige Varus- bzw. Valgusstellung des Knies zu ermöglichen, ebenso wie eine Aufnahme der Seitbewegungen als auch gegebenenfalls eine Korrektur von pathologischen Fehlstellungen, ist eine Ausführungsform vorgesehen, bei welcher zumindest einer der überlappenden Bereiche zumindest eines der Gelenkschenkeln mit dem daran anschließenden Abschnitt des Gelenkschenkels über ein monozentrisches Gelenk verbunden ist, dessen Achse im wesentlichen parallel zu der durch die Winkelbewegung der beiden Gelenkschenkel definierten Ebene orientiert ist.

Wenn gemäß einem weiteren optionalen Erfindungsmerkmal zumindest einer der überlappenden Bereiche zumindest eines der Gelenkschenkeln mit dem daran anschließenden Abschnitt des Gelenkschenkels über zwei Gelenks-Elemente miteinander verbunden sind, die eine Rotationsbewegung zwischen Stützkorpus und Orthesen-Gelenk bzw. Gelenk der Kniegelenk-Stütze um die Achse des Unterschenkels ermöglichen, kann die bei gebeugtem Knie physiologisch mögliche Drehung des Unterschenkels um seine Längsachse stattfinden.

Ein weiteres optionales Merkmale sieht vor, dass zumindest einer der überlappenden Bereiche zumindest eines der Gelenkschenkeln mit dem daran anschließenden Abschnitt des Gelenkschenkels über ein Längenverstellsystem verbunden ist, das eine Abstandsveränderung zwischen dem den anderen Gelenkschenkel überlappenden Abschnitt und dem übrigen Teil des Gelenkschenkels ermöglicht. Der Vorteil der Längenverstellung besteht darin, die Belastung, welche die Femurkondylen auf das Tibiaplateau ausüben, zu reduzieren.

Zur Lösung der gestellten Aufgabe ist auch eine Knie-Orthese mit zumindest zwei dem Unterschenkel zugeordneten ersten Gelenkschenkeln und zumindest zwei dem Oberschenkel zugeordneten zweiten Gelenkschenkeln geeignet, welche jeweils über ein Gelenk gemäß einem der vorhergehenden Absätze miteinander verbunden sind, wobei sich die beiden Gelenke bzw. die Drehpunkte in unterschiedlichen Höhen befinden, um beispielsweise eine Anpassung an die jeweilige Varus- bzw. Valgusstellung des Knies zu ermöglichen.

In der nachfolgenden Beschreibung soll die Erfindung anhand von bevorzugten, jedoch nicht einschränkenden Ausführungsbeispielen und unter Bezugnahme auf die beigefügten Zeichnungen näher erläutert werden.

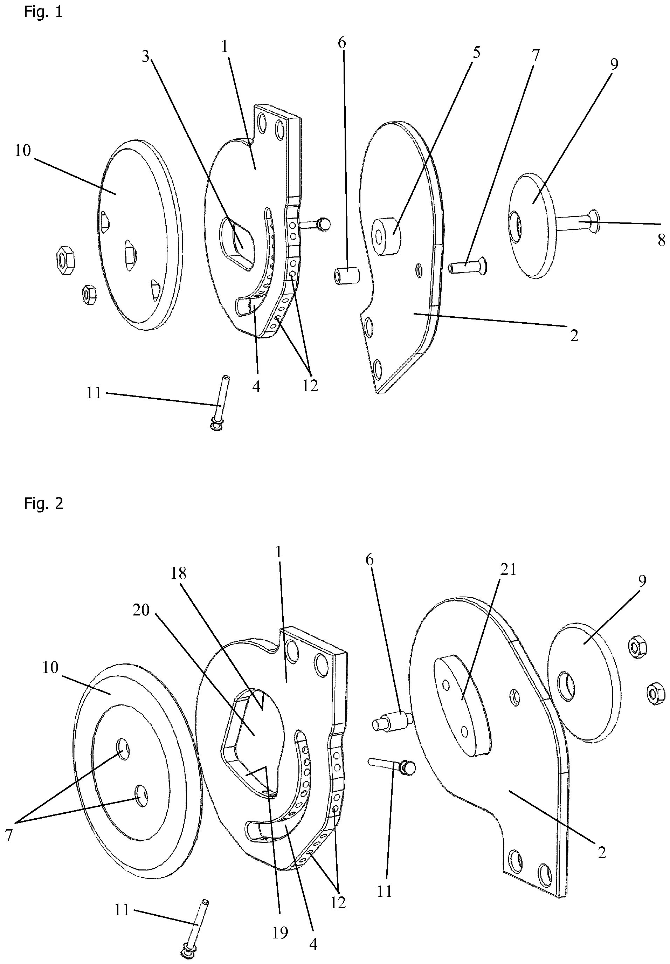

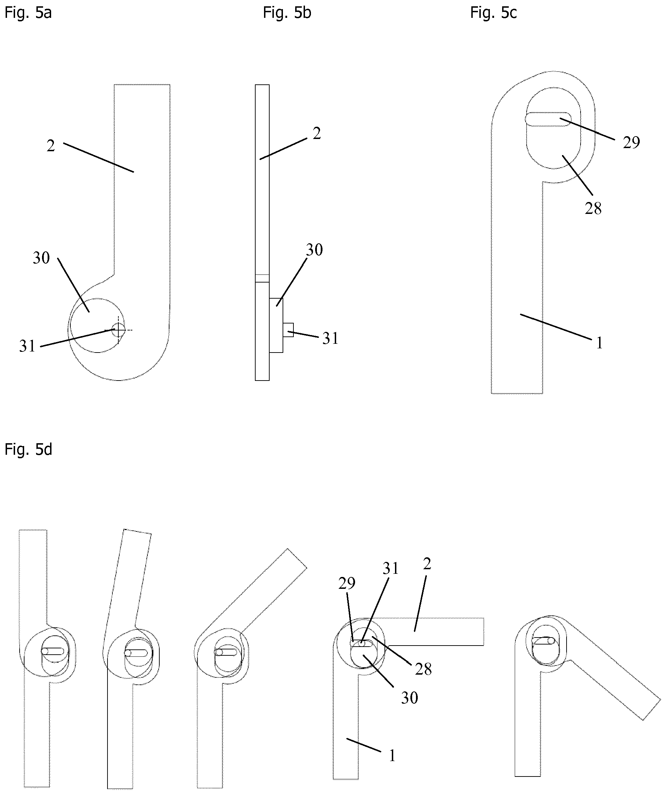

Dabei zeigen die Fig. 1 bis 4 jeweils unterschiedliche Ausführungsformen eines Gelenks in Explosionsdarstellung und die Fig. 5 zeigt eine erfindungsgemäßen Ausführungsform in der Drauf- und Seitenansicht sowie in verschiedenen Schwenkpositionen.

Die Hauptfunktion des Gelenks gemäß der vorliegenden Erfindung ist die Nachempfindung der Kinematik des menschlichen Knies, um bei Anwendung des Gelenks in Kniegelenks-Prothesen, -Orthesen oder -Stützen ein derart variables Rotationszentrum zu erhalten, dass keinerlei Spannungen in der Prothese oder Orthese entstehen können, welche für den Träger bzw. Patienten unangenehm empfunden werden oder die Beweglichkeit des Beins behindern.

Dazu umfasst das Gelenk zumindest einen dem Unterschenkel zugeordneten ersten Gelenkschenkel 1 und zumindest einen dem Oberschenkel zugeordneten zweiten Gelenkschenkel 2, welche mit ihren Enden überlappend verbunden sind. Bei der in Fig. 1 darstellten Ausführungsform, die kein Gegenstand der Erfindung ist, sind im ersten Gelenkschenkel 1 zwei Kurven 3, 4 ausgearbeitet. Eine erste Kurve 3 ist als im Wesentlichen gerader Schlitz ausgebildet, während eine zweite, gekrümmte Kurve 4 um den Schlitz 3 als Zentrum herum ausgearbeitet ist. Am zweiten Gelenkschenkel 2 sind zwei Führungselemente 5, 6 vorgesehen, wobei jeweils ein Führungselement 5 bzw. 6 in eine der Kurven 3 bzw. 4 eingreift und darin entlang dieser Kurven 3, 4 verschiebbar ist. Im Zuge der Beugebewegung der beiden Gelenkschenkel 1, 2 zueinander wird das in die zweite Kurve 4 eingreifende Führungselement 6 entsprechend der Krümmung der Kurve 4 geführt, welche Bewegung des Führungselementes 6 entlang der gekrümmten Bahn den Drehpunkt der beiden Gelenkschenkel 1, 2 bis zu einem Winkel zwischen den Gelenkschenkeln 1, 2 von 25° im Wesentlichen stationär hält.

Bei Fortsetzung der Beugebewegung der Gelenkschenkel 1, 2 bewirkt dann die weitere vorzugsweise progressive Bewegung des Führungselementes 6 im Schlitz 4 auch eine zunehmende Verschiebung des anderen Führungselementes 5 im geraden Schlitz 3. Die bei stationärem Drehpunkt alleinige Drehbewegung geht dabei bei zunehmendem Winkel zwischen den Gelenkschenkeln 1, 2 in eine kombinierte Roll- und Gleitbewegung in der durch die beiden Gelenkschenkel 1, 2 definierten Ebene über, die den Drehpunkt vorzugsweise progressiv im Wesentlichen radial zur Achse des ersten Gelenkschenkels 1 in den zwischen den beiden Gelenkschenkeln 1, 2 eingeschlossenen Quadranten führt.

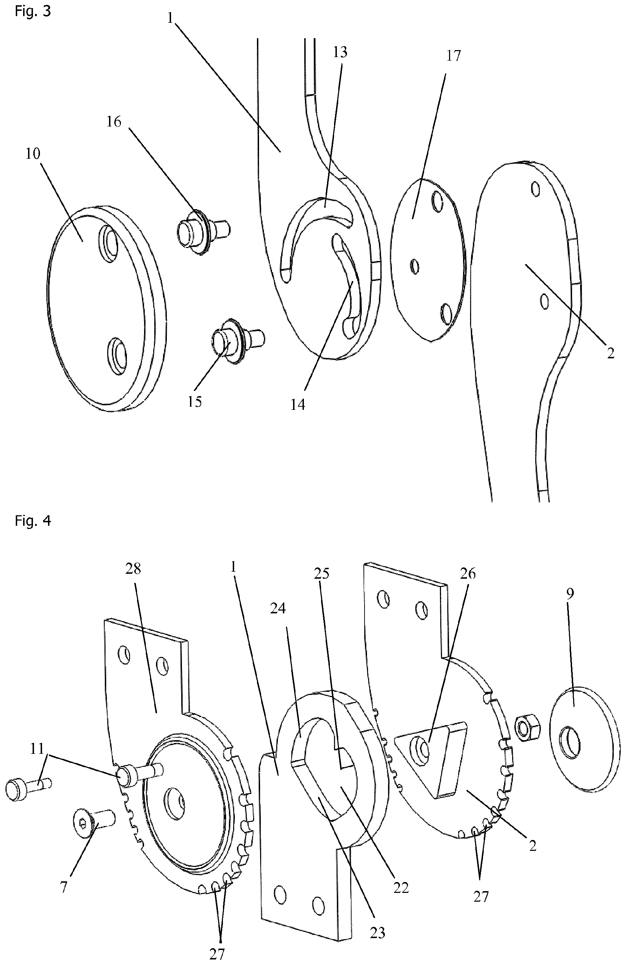

In einer Kniegelenks-Orthese werden jeweils zwei der erfindungsgemäßen Gelenke eingesetzt, wobei diese entweder einteilig mit sich entlang des Ober- bzw. Unterschenkel des Trägers erstreckenden Längselementen ausgeführt sein können - siehe dazu beispielsweise Fig. 3 - oder wie in den dargestellten Ausführungsbeispielen der Fig. 1, 2 und 4 auch mit den Gelenkschenkeln 1, 2 durch beispielsweise Verschraubung oder Vernietung verbunden sein können. Die Bewegung der Gelenkschenkel 1, 2 zueinander wird durch die interaktiven Kurven 3, 4 derart gesteuert, dass sich der dem Oberschenkel zugeordnete Gelenksschenkel 2 relativ zu dem dem Unterschenkel zugeordneten Gelenkschenkel 1 von anterior (von der vorderen Seite des Knies betrachtet) nach hinten (posterior) verschiebt, und zwar so, dass bis zu einem Beugungswinkel des Knies (Flexion) von ca. 25 Grad vorwiegend eine Drehbewegung ohne sichtbare Verschiebung des Drehzentrums in Richtung posterior (nach hinten) stattfindet, welche anschließend in eine gleichzeitige Roll und Gleitbewegung mit einer progressiven horizontalen Verschiebung des Drehpunktes zwischen den beiden Gelenkschenkeln 1, 2 nach hinten (posterior) übergeht. Der bis zum Ende der Rotation entstehende Beugungswinkel der erfindungsgemäßen Gelenke ist vorzugsweise mit ca. 120 Grad begrenzt, könnte aber auch bis zum maximalen Beugungswinkel eines Knies von ca. 135 Grad gehen.

Die Führungselemente 5, 6 können als separate Elemente ausgeführt sein, wie in Fig. 1 das Führungselement 6, die mittels beispielsweise Schrauben 7 mit den Gelenkschenkeln verbunden werden. Sie können aber auch einteilig mit den Gelenkschenkeln 1, 2 ausgebildet sein, wie im dargestellten Beispiel der Fig. 1 das Führungselement 5, durch welches hindurch eine Schraube 8 geführt ist, die eine erste Abdeckscheibe 9 mit einer zweiten Abdeckscheibe 10 verbindet und so das Gelenk zusammenhält.

Nachdem oftmals eine Begrenzung der Beweglichkeit zwischen dem Oberschenkel und dem Unterschenkel des Trägers, insbesondere von Orthesen, erforderlich ist, sind zwei Stifte 11 oder ähnliche Elemente vorgesehen, die durch radiale Bohrungen 12 in einem der Gelenkschenkeln 1, 2 gesteckt und darin fixiert werden können. Je nach Platzierung der Stifte 11 ist die Bewegung des Führungselementes 6 im Schlitz 4, und damit auch der Umfang der möglichen Beugebewegung der Gelenkschenkel 1, 2 zueinander, begrenzt.

Ebenfalls zwei Kurven in Schlitzform 13, 14 weist die Ausführungsform des Gelenks gemäß Fig. 3 auf. Hier sind beide im Gelenkschenkel 1 ausgearbeiteten Langschlitze 13, 14 um den Drehpunkt der Gelenkschenkel 1, 2 als Krümmungsmittelbereich herum gekrümmt ausgeführt. In jedem Schlitz 13, 14 ist als Führungselement jeweils ein Bolzen 15, 16 geführt, welche Bolzen 15, 16 mit dem zweiten Gelenkschenkel 2 verbunden sind. Im Zuge der Drehung wird durch die Anordnung und die sich entlang des Umfangs verändernde Krümmung interaktiv die physiologische Dreh- und progressive Gleitbewegung der beiden Gelenkschenkel 1, 2 zueinander bewirkt, wie bereits oben im Zusammenhang mit Fig. 1 beschrieben ist.

Die beiden Bolzen 15, 16 erstrecken sich vorzugsweise durch die Schlitze 13, 14 hindurch bis auf die gegenüberliegenden Seite des Gelenkschenkels 1 und verbinden den zweiten Gelenkschenkel 2 wiederum mit einer Abdeckplatte 10, die die Gelenkschenkel 1 und 2 zusammenhält. Eine reibungsvermindernde Zwischenplatte 17 erleichtert die Relativbewegung der beiden Gelenkschenkel 1, 2.

Um eine Begrenzung des Umfanges der möglichen Beugebewegung der Gelenkschenkel 1, 2 zueinander zu ermöglichen, ist eine Anordnungvon Bohrungen 17, zumindest je einem Stift (nicht dargestellt) pro Gelenkschenkel 1, 2 und einem nasenartigen Vorsprung 1a, 2a an jedem der Gelenkschenkel 1, 2 vorgesehen. Die Reihe der Bohrungen 27 verläuft im Gelenkschenkel 1 im Wesentlichen parallel zum Schlitz 13 und im Gelenkschenkel 2 im Wesentlichen spiegelverkehrt dazu. Wenn nun in zumindest einer der Bohrungen 27 ein Stift eingesetzt ist, kommt im Zuge der gegenseitigen Bewegung der Gelenkschenkel 1, 2 relativ zueinander der Vorsprung 1a, 2a zum Anschlag an diesem Stift und sperrt so die weitere Relativbewegung der Gelenkschenkel 1, 2. Diese Wirkung kann bei zumindest zwei vorhandenen Stiften sowohl für die Beuge- als auch die Streckbewegung erzielt werden. Wenn sogar pro Gelenkschenkel 1, 2 jeweils zwei Stifte vorgesehen sind, kann die Festigkeit und damit die Sicherheit noch weiter erhöht werden. Bei der Ausführungsform der Fig. 2 sind wiederum zwei Führungskurven vorgesehen. Diese beiden Führungskurven 18, 19 begrenzen zusammen einen Hohlraum 20, der im Gelenkschenkel 1 ausgearbeitet ist. In diesen Hohlraum 20 ragt ein nockenförmiger Führungskörper 21, der mit jeweils einem seiner beiden äußersten Abschnitten an jeweils einer Führungskurve 18, 19 geführt ist. Weiters ist auch als weitere Kurve ein Schlitz 4 um den Hohlraum 20 als Zentrum herum gekrümmt ausgearbeitet. In diesem gekrümmten Schlitz 4 ist ein am Gelenkschenkel 2 befestigtes Führungselement 6 beweglich geführt und darin verschiebbar, um damit und in Kombination mit der Wirkung des Führungskörpers 21 im Hohlraum 20 wiederum die oben bereits beschriebene stationäre und anschließend in die kombinierte Roll- und Gleitbewegung übergehende Bewegung des Drehpunktes zwischen den Gelenkschenkeln 1, 2 zu bewirken.

Der Zusammenhalt des Gelenks der Fig. 2 wird durch zwei Schrauben 7 bewirkt, die durch die Abdeckplatte 10 hindurch in den Führungskörper 21 eingeschraubt werden. Die andere Seite des Gelenks kann wieder durch eine Abdeckplatte 9 geschützt sein. Auch die Anordnung aus Stiften 11 und radialen Bohrungen 12 zur Begrenzung der Bewegung des Führungselementes 6 im Schlitz 4 damit auch der Begrenzung des Umfanges der möglichen Beugebewegung der Gelenkschenkel 1, 2 zueinander kann vorgesehen sein.

In der Ausführungsform der Fig. 4 ist ein Hohlraum 22 im Gelenkschenkel 1 ausgearbeitet, welcher durch drei Führungskurven 23, 24, 25 begrenzt ist. In diesen Hohlraum 22 greift ein dreieckiger Führungskörper 26 ein, welchem mit dem anderen Gelenkschenkel 2 fest verbunden oder daran ausgebildet ist, um durch Entlanggleiten der drei äußersten Bereiche des Führungskörpers 26 entlang jeweils einer der drei Führungskurven 23, 24, 25 die physiologische Dreh- und Gleitbewegung der beiden Gelenkschenkel 1, 2 zueinander zu bewirken.

Auch bei der Ausführungsform der Fig. 4 ist, wie im Fall der Ausführungsform der Fig. 3, über eine Anordnung aus Stiften 11 und Bohrungen 27 die Begrenzung der Bewegung des Führungskörpers 26 im Hohlraum 22 damit auch der Begrenzung des Umfanges der möglichen Beugebewegung der Gelenkschenkel 1, 2 zueinander möglich. Die Bohrungen 27 sind nun allerdings ganz zum äußeren Rand des Gelenkschenkels 2 sowie zum Rand einer dem Gelenkschenkel 2 in der Form im Wesentlichen entsprechenden Abdeckplatte 28 verschoben, so dass sie eigentlich radial nach außen offene Einkerbungen in diesen Bauteilen 2, 28 darstellen.

Die optimale physiologische Anpassung der Relativbewegung der Gelenkschenkel 1, 2 im Zuge der Beuge- und/oder Streckbewegung des Beins kann auch durch eine Konstruktion wie in den Fig. 5a bis 5c dargestellt erzielt werden, bei der im ersten Gelenkschenkel 1 eine erste, im Wesentlichen gerade, nutartige Ausnehmung 28 im Wesentlichen in Richtung der Längsachse des Gelenkschenkels 1 orientiert ausgearbeitet ist. Senkrecht dazu ist weiters ein im Wesentlichen gerader Längsschlitz 29 mit geringerer Breite als die Ausnehmung 28 im Gelenkschenkel 1 ausgearbeitet, welcher Längsschlitz 29 im Wesentlichen senkrecht auf die Ausnehmung 28 orientiert ist.

Am zweiten Gelenkschenkel 2 hingegen sind zwei im Wesentlichen kreisrunde Führungselemente 30, 31 angesetzt. Das unmittelbar am Gelenkschenkel 2 anliegende Führungselement 30 ist exzentrisch angeordnet und weist einen Durchmesser auf, welcher der Breite der Ausnehmung 28 entspricht, so dass dieses Führungselement 30 sich in der Ausnehmung 28 drehen als auch diese Ausnehmung 28 entlang gleiten kann. Aufgesetzt auf dem ersten Führungselement 30 ist ein zweites Führungselement 31, welches einen geringeren, der Breite des Längsschlitzes 29 im ersten Gelenkschenkel 1 entsprechenden Durchmesser aufweist und im Wesentlichen im Zentrum des mit dem anderen Gelenkschenkel 1 überlappenden Endes des die Führungselemente 30, 31 tragenden Gelenkschenkels 2 angeordnet ist. Dieses Führungselement 31 kann sich daher im Längsschlitz 29 frei drehen und in Längsrichtung des Längsschlitzes 29 gleiten.

Wie in den in Fig. 5d dargestellten unterschiedlichen Stadien der Beugebewegung zu erkennen ist, findet durch die nur leichte Verschiebung des Führungselementes 30 in der Ausnehmung 28 bei im Wesentlichen stationärem Führungselement 31 zu Beginn der Beugebewegung vorwiegend eine Drehbewegung des dem Oberschenkel zugeordneten Gelenkschenkels 2 relativ zum dem Unterschenkel zugeordneten Gelenkschenkel 1 statt (siehe die beiden Stadien links in Fig. 5d). Bis zu einem Beugungswinkel des Knies (Flexion) von ca. 25 Grad findet somit vorwiegend eine Drehbewegung ohne sichtbare Verschiebung des Drehzentrums in Richtung posterior (nach hinten) statt.

Anschließend (siehe dazu die mittlere Abbildung der Fig. 5d und die zweite Abbildung von rechts), bei weiterer Verschiebung des Führungselementes 30 in der Ausnehmung 28 findet eine Kombination von Drehbewegung und Verschiebung des Drehzentrums in Richtung posterior statt, die mit der Verschiebung des Führungselementes 31 im Längsschlitz 29 einhergeht. Ab den oben genannten ca. 25 Grad Beugungswinkel findet eine gleichzeitige Roll- und Gleitbewegung mit einer progressiven horizontalen Verschiebung des Drehpunktes zwischen den beiden Gelenkschenkeln 1, 2 nach hinten (posterior) statt. Ganz zum Schluss der Beugebewegung, wobei der Beugungswinkel auch hier vorzugsweise mit ca. 120 Grad begrenzt ist, aber auch bis zum maximalen Beugungswinkel eines Knies von ca. 135 Grad gehen könnte, erfolgt im Wesentlichen ausschließlich eine Verschiebung nach hinten des oberen Gelenkschenkels 2 gegenüber dem unteren Gelenkschenkel (siehe dazu die beiden Abbildungen ganz rechts in Fig. 5d).

## Patentansprüche

1. Gelenk für Kniegelenks-Orthesen, -Prothesen bzw. -Stützen, mit zumindest einem dem Unterschenkel zugeordneten ersten Gelenkschenkel (1) und zumindest einem dem Oberschenkel zugeordneten zweiten Gelenkschenkel (2), welche mit ihren Enden derart überlappend verbunden sind, dass bei zunehmendem Winkel zwischen den Gelenkschenkeln (1, 2) eine kombinierte Roll- und Gleitbewegung in der durch die beiden Gelenkschenkel definierten Ebene bewirkt ist, **dadurch gekennzeichnet, dass** im ersten Gelenkschenkel (1) eine erste, im Wesentlichen gerade, nutartige Ausnehmung (28) im Wesentlichen in Richtung der Längsachse des Gelenkschenkels (1) orientiert ausgearbeitet ist, dass weiters im ersten Gelenkschenkel (1) ein im Wesentlichen gerader Längsschlitz (29) mit geringerer Breite als die Ausnehmung (28) und im Wesentlichen senkrecht auf diese Ausnehmung (28) ausgearbeitet ist, und dass am zweiten Gelenkschenkel (2) zwei im Wesentlichen kreisrunde Führungselemente (30, 31) angesetzt sind, wobei das dem Gelenkschenkel (2) unmittelbar anliegende Führungselement (30) exzentrisch angeordnet und mit einem der Breite der Ausnehmung (28) entsprechenden Durchmesser versehen ist, und wobei das zweite Führungselement (31) auf dem ersten Führungselement (30) aufgesetzt ist, einen der Breite des Längsschlitzes (29) entsprechenden Durchmesser aufweist und im Wesentlichen im Zentrum des mit dem anderen Gelenkschenkel (1) überlappenden Endes des Gelenkschenkels (2) angeordnet ist, wobei bei zunehmendem Winkel zwischen den Gelenkschenkeln (1, 2) eine kombinierte Roll- und Gleitbewegung des zweiten Gelenkschenkels (2) in der durch die beiden Gelenkschenkel definierten Ebene in einer Richtung bewirkt ist, welche im wesentlichen radial zur Achse des ersten Gelenkschenkels (1) in den zwischen den beiden Gelenkschenkel eingeschlossenen Quadranten führt, wobei der Drehpunkt der beiden Gelenkschenkel (1, 2) für einen Winkel zwischen den Gelenkschenkeln zwischen 0 und 25°im Wesentlichen stationär bleibt.

2. Gelenk nach Anspruch 1, **dadurch gekennzeichnet, dass** zumindest einer der überlappenden Bereiche zumindest eines der Gelenkschenkeln (1, 2) mit dem daran anschließenden Abschnitt des Gelenkschenkels über ein monozentrisches Gelenk verbunden ist, dessen Achse im wesentlichen parallel zu der durch die Winkelbewegung der beiden Gelenkschenkel (1, 2) definierten Ebene orientiert ist.

3. Gelenk nach Anspruch 1 oder 2, **dadurch gekennzeichnet, dass** zumindest einer der überlappenden Bereiche zumindest eines der Gelenkschenkeln (1, 2) mit dem daran anschließenden Abschnitt des Gelenkschenkels über zwei Gelenks-Elemente miteinander verbunden ist, die eine Rotationsbewegung zwischen Stützkorpus und Orthesen-Gelenk bzw. Gelenk der Kniegelenk-Stütze um die Achse des Unterschenkels ermöglichen.

4. Gelenk nach einem der Ansprüche 1 bis 3, **dadurch gekennzeichnet, dass** zumindest einer der überlappenden Bereiche zumindest eines der Gelenkschenkeln (1, 2) mit dem daran anschließenden Abschnitt des Gelenkschenkels über ein Längenverstellsystem verbunden ist, das eine Abstandsveränderung zwischen dem den anderen Gelenkschenkel überlappenden Abschnitt und dem übrigen Teil des Gelenkschenkels ermöglicht.

5. Knie-Orthese mit zumindest zwei dem Unterschenkel zugeordneten ersten Gelenkschenkeln (1) und zumindest zwei dem Oberschenkel zugeordneten zweiten Gelenkschenkeln (2), welche jeweils über ein Gelenk gemäß einem der Ansprüche 1 bis 4 miteinander verbunden sind.

6. Knie-Orthese nach Anspruch 5, **dadurch gekennzeichnet, dass** sich die beiden Gelenke bzw. die Drehpunkte in Gebrauch in unterschiedlichen Höhen befinden.

## Claims

1. A joint for knee joint orthotics, prosthetics and supports, with at least one first joint leg (1) associated to the lower leg and at least one second joint leg (2) associated to the thigh, which are overlappingly connected with their ends, in such a way that with an increasing angle between the joint legs (1, 2) a combined rolling and sliding movement in the plane defined by the two joint legs is obtained, **characterized in that,** in the first joint leg (1), a first essentially straight slot-like recess (28) is formed, which is directed essentially in the direction of the longitudinal axis of the joint leg (1), that further in the first joint leg (1) an essentially straight longitudinal slit (29) is formed, which has a smaller width with respect to the recess (28) and is essentially perpendicular to this recess (28), and that on the second joint leg (2) two essentially circular guide elements (30, 31) are set, wherein the guide element (30) directly abutting against the joint leg (2) is eccentrically positioned and is provided with a diameter corresponding to the width of the recess (28), and wherein the second guide element (31) is set on the first guide element (30), has a diameter corresponding to the width of the longitudinal slit (29) and is positioned essentially at the center of the end of the joint leg (2) overlapping the other joint leg (1), wherein, with an increasing angle between the joint legs (1, 2) a combined rolling and sliding movement of the second joint leg (2) in the plane defined by both joint legs is obtained, which essentially leads radially towards the axis of the first joint leg (1) in the quadrant enclosed between the two joint legs, wherein the fulcrum of the two joint legs (1, 2) remains substantially stationary for an angle between the joint legs between 0 and 25°.

2. The joint according to claim 1, **characterized in that** at least one of the overlapping regions of at least one of the joint legs (1, 2) is connected to the adjoining portion of the joint leg through a monocentric joint, the axis of which is directed essentially in parallel to the plane defined by the angular movement of the two joint legs (1, 2).

3. The joint according to claim 1 or 2, **characterized in that** at least one of the overlapping regions of at least one of the joint legs (1, 2) is connected to the adjoining portion of the joint leg via two joint elements, which allow a rotational movement between the support body and the orthotics joint or joint of the knee joint support around the axis of the lower leg.

4. The joint according to any of claims 1 to 3, **characterized in that** at least one of the overlapping regions of at least one of the joint legs (1, 2) is connected to the adjoining portion of the joint leg via a longitudinal displacement system, which provides a modification of the distance between the portion overlapping the other joint leg and the remaining part of the joint leg.

5. A knee orthotics with at least two first joint legs (1) associated to the lower leg and at least two second joint legs (2) associated to the thigh, which are connected to each other via a joint according to any of claims 1 to 4.

6. The knee orthotics according to claim 5, **characterized in that** the two joints or their fulcra are positioned, in use, at different heights.

## Revendications

1. Articulation pour orthèses, prothèses ou supports d'articulation du genou, comprenant au moins une première branche d'articulation (1) associée au bas de la jambe et au moins une deuxième branche d'articulation (2) associée à la cuisse, lesquelles branches sont reliés à chevauchement par leurs extrémités de telle sorte que, lorsque l'angle formé entre les branches d'articulation (1, 2) augmente, un mouvement combiné de roulement et de glissement se produit dans le plan défini par les deux branches d'articulation, **caractérisée en ce que,** dans la première branche d'articulation (1), un premier évidement (28) sensiblement rectiligne, analogue à une gorge, est formé de manière orientée sensiblement en direction de l'axe longitudinal de la branche d'articulation (1), **en ce qu'**une fente longitudinale (29) sensiblement rectiligne, d'une largeur inférieure à celle de l'évidement (28) et sensiblement perpendiculaire à cet évidement (28), est en outre ménagée dans la première branche d'articulation (1), et **en ce que** deux éléments de guidage (30, 31) sensiblement circulaires sont fixés à la deuxième branche d'articulation (2), l'élément de guidage (30) directement adjacent à la branche d'articulation (2) étant disposé de manière excentrique et étant pourvu d'un diamètre correspondant à la largeur de l'évidement (28), et le deuxième élément de guidage (31) étant placé sur le premier élément de guidage (30), ayant un diamètre correspondant à la largeur de la fente longitudinale (29) et étant disposé sensiblement au centre de l'extrémité de la branche d'articulation (2) qui chevauche l'autre branche d'articulation (1), un mouvement combiné de roulement et de glissement de la deuxième branche d'articulation (2) étant généré, lorsque l'angle formé entre les branches d'articulation (1, 2) augmente, dans le plan défini par les deux branches d'articulation dans une direction qui est sensiblement radiale par rapport à l'axe de la première branche d'articulation (1) dans le quadrant compris entre les deux branches d'articulation, le point de pivotement des deux branches d'articulation (1, 2) restant sensiblement stationnaire pour un angle formé entre les branches d'articulation étant compris entre 0 et 25°.

2. Articulation selon la revendication 1, **caractérisée en ce qu'**au moins une des zones de chevauchement d'au moins l'une des branches d'articulation (1, 2) est reliée à la partie adjacente de la branche d'articulation par une articulation monocentrique dont l'axe est orienté de manière sensiblement parallèle au plan défini par le mouvement angulaire des deux branches d'articulation (1, 2).

3. Articulation selon la revendication 1 ou 2, **caractérisée en ce qu'**au moins une des régions de chevauchement d'au moins une des branches d'articulation (1, 2) est reliée à la partie adjacente de la branche d'articulation par le biais de deux éléments d'articulation qui assurent un mouvement de rotation sur l'axe du bas de la jambe entre le corps de support et l'articulation d'une orthèse ou l'articulation du support d'articulation de genou.

4. Articulation selon l'une des revendications 1 à 3, **caractérisée en ce qu'**au moins une des zones de chevauchement d'au moins une des branches d'articulation (1, 2) est reliée à la partie adjacente de la branche d'articulation par le biais d'un système de réglage de longueur qui permet de modifier la distance entre la partie chevauchant l'autre branche d'articulation et la partie restante de la branche d'articulation.

5. Orthèse de genou comportant au moins deux premières branches d'articulation (1) associées au bas de la jambe et au moins deux deuxièmes branches d'articulation (2) associées à la cuisse, lesquelles branches sont reliées l'une à l'autre par une articulation selon l'une des revendications 1 à 4.

6. Orthèse de genou selon la revendication 5, **caractérisée en ce que** les deux articulations ou les points de pivotement se trouvent en utilisation à des hauteurs différentes.
